# EUROPEAN PATENT SPECIFICATION

(11) **EP 4 268 821 B1**
(45) Date of publication and mention of the grant of the patent: **02.12.2026**
(21) Application number: 21913909.4
(22) Date of filing: 15.12.2021
(51) Int. Cl.: A61K 9/16, A61K 31/4745, A61K 9/48, A61K 47/04, A61P 25/28, A61P 25/16

(54) **PHARMACEUTICAL COMPOSITION CONTAINING PYRROLOQUINOLINE QUINONE TRILITHIUM SALT NONAHYDRATE COMPOUND, CAPSULE, AND PREPARATION METHOD THEREFOR**
PHARMAZEUTISCHE ZUSAMMENSETZUNG MIT PYRROLOCHINOLINCHINONTRILITHIUMSALZ-NONHYDRATVERBINDUNG, KAPSEL UND HERSTELLUNGSVERFAHREN DAFÜR
COMPOSITION PHARMACEUTIQUE CONTENANT UN COMPOSÉ NONAHYDRATE DE SEL DE TRILITHIUM DE PYRROLOQUINOLÉINE QUINONE, CAPSULE ET SON PROCÉDÉ DE PRÉPARATION

(30) Priority: 28.12.2020 CN 202011573509
(43) Date of publication of application: 01.11.2023
(73) Proprietor: Shanghai Raising Pharmaceutical Co., Ltd., Pudong New District Shanghai 201321 (CN)
(72) Inventor: ZHANG, Huan, Shanghai 201321 (CN)
(74) Representative: Nederlandsch Octrooibureau
(86) International application number: PCT/CN2021/138255
(87) International publication number: WO 2022/143168

(56) References cited:
- EP-A1- 2 415 770
- EP-A1- 2 998 305
- CN-A- 101 851 234
- CN-A- 103 351 387
- CN-A- 112 274 513
- JP-A- 2011 126 812
- US-A1- 2015 203 488
- ZHAO LEI ET AL: "Beneficial synergistic effects of microdose lithium with pyrroloquinoline quinone in an Alzheimer's disease mouse model", NEUROBIOLOGY OF AGING, vol. 35, no. 12, 1 December 2014 (2014-12-01), US, pages 2736 - 2745, XP093218145, ISSN: 0197-4580, DOI: 10.1016/j.neurobiolaging.2014.06.003

## Description

The present application claims the priority of Chinese patent application No. 202011573509.7, entitled "pharmaceutical composition comprising pyrroloquinoline quinone trilithium salt nonahydrate compound, capsule, and preparation method therefor", filed before the China National Intellectual Property Administration on December 28, 2020.

### Technical Field

The present invention belongs to the technical field of pharmaceutical formulations, and particularly relates to a pharmaceutical composition comprising a pyrroloquinoline quinone trilithium salt nonahydrate compound, as well as a capsule comprising the composition and a preparation method therefor.

### Background Art

The compound represented by Formula (I) is pyrroloquinoline quinone trilithium salt nonahydrate compound, having a chemical name of 4,5-dioxo-4,5-dihydro-1H-pyrrolo[2,3-f]quinoline-2,7,9-tricarboxylic acid lithium salt nonahydrate compound, a molecular formula of C₁₄H₃Li₃N₂O₈·9H₂O, and a molecular weight of 510.14, and it is a promising candidate drug for the treatment of various neurodegenerative diseases such as Parkinson's disease and Alzheimer's disease.

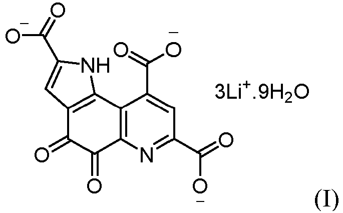

Chinese invention patent applications 200910048873.9 and 201310270885.2 respectively disclose a lithium salt form and a nonahydrate crystalline form of the pyrroloquinoline quinone compound. As described in Chinese invention patent application 201310270885.2, the crystalline form of the compound represented by Formula (I) has characteristic diffraction peaks at Power X-ray diffraction (PXRD) angles 2θ=6.2°±0.2°, 7.4°±0.2°, 7.9°±0.2° and 23.6°±0.2°, etc. It is also found that this nonahydrate compound has a crystal water content of up to 31.8%, is sensitive to temperature, loses the crystal water at 40°C, and rapidly and irreversibly loses water at a temperature higher than 60°C.

EP2998305A1 discloses a pharmaceutical composition comprising 1.4 mg crystalline pyrroloquinoline quinone tri-lithium salt nonahydrate, 116.2 mg microcrystalline cellulose and 2.4 mg magnesium stearate. The API has a X-ray powder diffraction pattern comprising characteristic diffraction peaks of 6.2°±0.2°, 7.4°±0.2°, 7.9°±0.2° and 23.6°±0.2°.

Due to the defects of the active pharmaceutical ingredient of pyrroloquinoline quinone trilithium salt nonahydrate in crystalline form such as easy aggregation, low bulk density, poor fluidity and easy adhesion to the surface of an equipment, it is necessary to increase the bulk density and fluidity by means of granulation, so as to ensure accuracy of the subsequent capsule filling process. It is found that the solvent removal process by drying in wet granulation leads to partial or complete loss of the crystal water of pyrroloquinoline quinone trilithium salt nonahydrate, resulting in the change of the crystalline form. Dry granulation does not require the use of a solvent, thereby avoiding the risk of crystalline form change caused by heating. However, pyrroloquinoline quinone trilithium salt nonahydrate is a metal salt comprising a high proportion of crystal water, and like many metal salts, it is easy to form a rigid and hard structure after being pressed. Dry granulation is a process of mixing a drug and excipient(s), rolling them into thin sheets, and then crushing them into granules. If the metal salt forms a thin sheet with a rigid structure after rolling, it not only makes the subsequent processing difficult, thereby decreasing the production efficiency, but also increase the temperature of the equipment after long-time rolling and repeated crushing, thereby affecting the stability of the crystalline form of pyrroloquinoline quinone trilithium salt nonahydrate. In addition, forced granulation makes the granules as prepared hard and also causes a decrease in the dissolution rate. If the amount of excipient(s) is increased to reduce the physical contact between metal salt raw material particles, thereby reducing the formation of a rigid structure, it would lead to an oversize of the final product at the same specification due to the excessive amount of excipient(s). For elderly patients with neurodegenerative diseases who have dysphagia and need lifelong medication, this can easily lead to both psychological and physiological medication barriers.

Therefore, currently there is no corresponding formulation containing a crystalline form of pyrroloquinoline quinone trilithium salt nonahydrate and a preparation process thereof, which can ensure the stability of the crystalline form of pyrroloquinoline quinone trilithium salt nonahydrate and good dissolution profile, and can ensure that each unit of the formulation has a high drug content while reducing the amount of excipient(s) employed as much as possible, making it convenient for elderly patients to take, thereby improving their medication compliance.

### Contents of the Invention

In order to solve the above technical problems, the invention provides a pharmaceutical composition comprising a pyrroloquinoline quinone trilithium salt nonahydrate compound, as well as a capsule comprising the composition and a preparation method therefor. The pharmaceutical composition, as well as the capsule comprising the composition and the preparation method therefor provided by the present invention can ensure that the crystalline form of pyrroloquinoline quinone trilithium salt nonahydrate has good stability and dissolution profile, and can ensure that each unit of the formulation has a high drug content while reducing the amount of excipient(s) employed as much as possible, making it convenient for elderly patients to take, and improving their medication compliance.

The present invention provides a pharmaceutical composition comprising a pyrroloquinoline quinone trilithium salt nonahydrate compound, wherein the pharmaceutical composition comprises:
1.0 part by weight of pyrroloquinoline quinone trilithium salt nonahydrate compound; and a pharmaceutically acceptable excipient;
the pharmaceutically acceptable excipient comprises:
   0.16 part by weight to 0.48 part by weight of colloidal silica;
   wherein the pharmaceutically acceptable excipient further comprises:
      1.0 part by weight to 5.0 parts by weight of additional pharmaceutically acceptable excipient(s);
      the additional pharmaceutically acceptable excipient is selected from one or more of starch, pregelatinized starch, microcrystalline cellulose, lactose, lactose starch complex, lactose cellulose complex, mannitol, mannitol starch complex, sorbitol, povidone, copovidone, hydroxypropylmethylcellulose, hydroxypropylcellulose, low-substituted hydroxypropylcellulose, croscarmellose sodium, crospovidone, magnesium stearate, sodium stearyl fumarate, pulvis talci, and stearic acid;
      wherein the pyrroloquinoline quinone trilithium salt nonahydrate compound is in a crystalline form, and the pyrroloquinoline quinone trilithium salt nonahydrate compound has an X-ray powder diffraction pattern comprising characteristic diffraction peaks at 20 diffraction angles of 6.2°±0.2°, 7.4°±0.2°, 7.9°±0.2° and 23.6°±0.2°.

Optionally, the colloidal silica is of 0.2 part by weight to 0.4 part by weight; and the additional pharmaceutically acceptable excipient is of 2.0 parts by weight to 4.0 parts by weight.

Optionally, the additional pharmaceutically acceptable excipient comprises: 0.6 part by weight to 4 parts by weight of pregelatinized starch; 0.36 part by weight to 0.96 part by weight of microcrystalline cellulose; and 0 part by weight to 0.06 part by weight of magnesium stearate.

Optionally, the colloidal silica can be added through an internal addition method or an internal-external addition method; and the additional pharmaceutically acceptable excipient can be added through an internal addition method or an internal-external addition method.

Optionally, when the colloidal silica is added through the internal-external addition method, the weight ratio of the internally added colloidal silica to the externally added colloidal silica is greater than 3:1; and when the additional pharmaceutically acceptable excipient is added through the internal-external addition method, the weight ratio of the internally added additional pharmaceutically acceptable excipient to the externally added additional pharmaceutically acceptable excipient is greater than 3:1.

Optionally, the additional pharmaceutically acceptable excipient which can be added through the internal addition method is selected from one or more of starch, lactose, microcrystalline cellulose, mannitol, croscarmellose sodium, copovidone, and magnesium stearate; and the additional pharmaceutically acceptable excipient which can be added through the external addition method is selected from one or more of microcrystalline cellulose, croscarmellose sodium, and magnesium stearate.

The present invention further provides a capsule comprising a pyrroloquinoline quinone trilithium salt nonahydrate compound, wherein the capsule comprising the pyrroloquinoline quinone trilithium salt nonahydrate compound comprises the pharmaceutical composition of the present invention.

Optionally, the capsule material can be selected from any one of gelatin capsule, hydroxypropylmethylcellulose capsule, pullulan polysaccharide capsule, and starch capsule.

Optionally, the capsule material is hydroxypropylmethylcellulose capsule.

Optionally, in the capsule formulation, each capsule comprises 5 mg to 100 mg of pyrroloquinoline quinone trilithium salt nonahydrate compound.

Optionally, each capsule comprises 20 mg to 75 mg of pyrroloquinoline quinone trilithium salt nonahydrate compound.

Furthermore, the present invention further provides a method for preparing a capsule comprising a pyrroloquinoline quinone trilithium salt nonahydrate compound, which is employed for preparing the capsule comprising the pyrroloquinoline quinone trilithium salt nonahydrate compound of the present invention, and the preparation method is a dry granulation method for preparing a capsule.

Optionally, when the pharmaceutically acceptable excipient is added through the internal addition method, the preparation method comprises:
weighing the active pharmaceutical ingredient (API) of the pyrroloquinoline quinone trilithium salt nonahydrate compound and the pharmaceutically acceptable excipient according to the formulation ratios;
mixing the pyrroloquinoline quinone trilithium salt nonahydrate compound API with the pharmaceutically acceptable excipient;
performing dry granulation on the mixed pyrroloquinoline quinone trilithium salt nonahydrate compound API and the pharmaceutically acceptable excipient, firstly rolling the mixture into thin sheets, and then granulating into granules; and
performing capsule filling with the granules to obtain the capsule.

Optionally, when the pharmaceutically acceptable excipient is added through the internal-external addition method, the preparation method comprises:
weighing the pyrroloquinoline quinone trilithium salt nonahydrate compound API and the pharmaceutically acceptable excipient according to the formulation ratios;
mixing the pyrroloquinoline quinone trilithium salt nonahydrate compound API and the pharmaceutically acceptable excipient for internal addition;
performing dry granulation on the mixed pyrroloquinoline quinone trilithium salt nonahydrate compound API and the pharmaceutically acceptable excipient for internal addition, firstly rolling the mixture into thin sheets, and then granulating into granules;
mixing the granules obtained by the dry granulation with the pharmaceutically acceptable excipient for external addition; and
performing capsule filling with the granules mixed with the pharmaceutically acceptable excipient for external addition, to obtain the capsule.

Compared with the prior art, the pharmaceutical composition comprising the pyrroloquinoline quinone trilithium salt nonahydrate compound, the capsule comprising the composition and the preparation method therefor provided by examples of the present invention have the following advantages:
The pharmaceutical composition comprising the pyrroloquinoline quinone trilithium salt nonahydrate compound provided by the present invention employs a proper proportion of colloidal silica and a proper proportion of an additional pharmaceutically acceptable excipient, utilizing the high specific surface area and low density of the colloidal silica (which mean that a high proportion of air is comprised), together with the dilution effect of the additional pharmaceutically acceptable excipient, thereby effectively alleviating the defect that the pyrroloquinoline quinone trilithium salt nonahydrate compound is prone to form a rigid thin sheets which are not easy to granulate in dry granulation. The thin sheets as prepared by dry granulation are loose and easy to granulate, and thus the production efficiency and yield are improved, making it possible for commercial production scale-up. Meanwhile, due to the large specific surface area and good anti-adhesion effect, the colloidal silica can solve the problems of easy aggregation and adhesion to the surface of an equipment.

In addition, the pyrroloquinoline quinone trilithium salt nonahydrate capsule prepared by the composition and capsule preparation method therefor of the invention can make the crystalline form of the pyrroloquinoline quinone trilithium salt nonahydrate compound have good stability and dissolution profile. Experimental results show that the capsule can be subjected to long-term storage at a temperature of 30°C, and maintains a good dissolution profile at 25°C, 60% relative humidity and 30°C, 65% relative humidity after 6 months in both of the long-term and accelerated tests, no related substances are detected, and the PXRD characteristic peaks and geometric topology patterns remain consistent with those on Day 0, indicating that the capsule comprising the pharmaceutical composition prepared by the process of the invention has good chemical stability. Moreover, due to the proper proportion of excipients used, excessive use of excipients is avoided, making the capsule as prepared suitable for elderly patients to swallow, convenient for elderly patients to take, thereby improving their medication compliance.

### Description of Figures

FIG 1 shows the microscopic observation result of pyrroloquinoline quinone trilithium salt nonahydrate compound API;
FIG 2 shows the dissolution profiles of capsule formulations No.1 to No.4 in Example 1;
FIG 3 shows the dissolution profiles of capsule formulations No.5 to No.8 in Example 2;
FIG 4 shows that the dissolution profiles of capsule formulations No.9 to No.12 in Example 3;
FIG 5 shows the dissolution profiles of capsule formulations No.13 to No.16 in Example 4;
FIG 6 shows the dissolution profiles of capsule formulations No.1 to No.2 in Comparative Example 1;
FIG 7 shows the dissolution profiles of capsule formulations No.3 to No.6 in Comparative Example 2;
FIG 8 shows the dissolution profiles of capsule formulations No.7 to No.10 in Comparative Example 3;
FIG 9 shows the dissolution profiles of capsule formulation No.3 in Example 1 and capsule formulation No.7 in Example 2 before and after the stability test for 6 months;
FIG 10 shows the PXRD measurement results of capsule formulation No.3 in Example 1 and capsule formulation No.7 in Example 2 before and after the 6-month test.

### Mode of Carrying Out the Invention

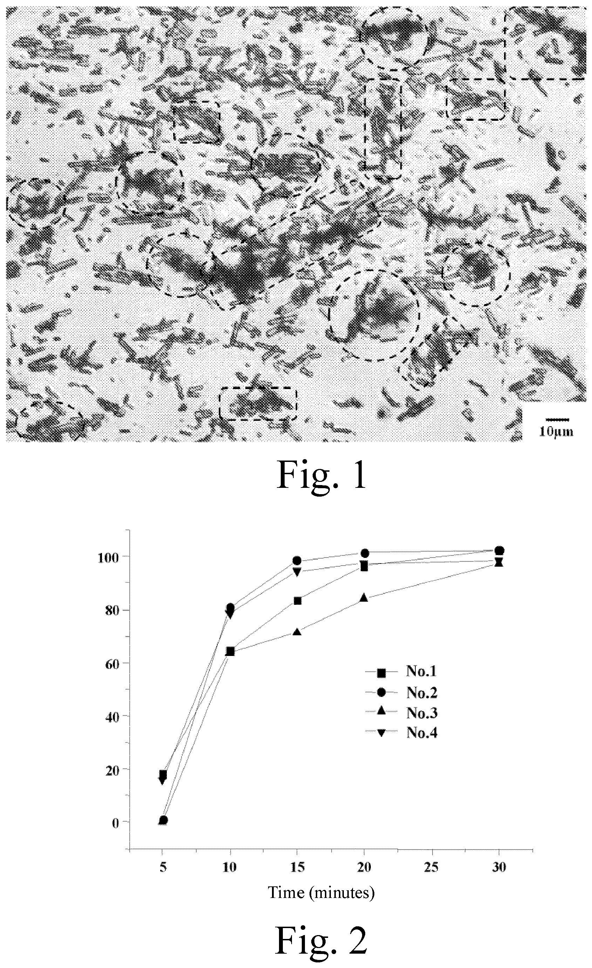

As described in the background art, the pyrroloquinoline quinone trilithium salt nonahydrate compound has a crystal water content of up to 31.8%, is sensitive to temperature, loses the crystal water at a temperature higher than 40°C, and rapidly and irreversibly loses water at a temperature higher than 60°C. The pyrroloquinoline quinone trilithium salt nonahydrate compound API is in a short rod-shaped shape, and the API particles are prone to aggregate (the microscopic observation result is shown in FIG 1, and the aggregated API particles are shown in the dotted circles). During the formulation development process, it is found that the API has defects such as low bulk density, poor fluidity, and easy adhesion to the surface of an equipment, etc.

Upon further investigation, the inventor found that due to the easy aggregation of pyrroloquinoline quinone trilithium salt nonahydrate compound, a high proportion of excipients are needed for dilution and dispersion. However, if the amount of excipients employed is too large, it may lead to a larger volume of the formulation and affect swallowability (the indication mainly targets elderly patients). The pyrroloquinoline quinone trilithium salt nonahydrate compound is a metal lithium salt. If the proportion of excipients in a formulation is low and the drug is not fully diluted and dispersed, thin sheets obtained by rolling are hard and difficult to crush (a common property shared by many metal salts), and if the thin sheets are crushed repeatedly at a high speed, friction heating will also affect the stability of the hydrate.

In order to solve the above technical problems, the present invention provides a pharmaceutical composition comprising the pyrroloquinoline quinone trilithium salt nonahydrate compound. It employs a proper proportion of colloidal silica and a proper proportion of an additional pharmaceutically acceptable excipient, utilizing the high specific surface area and low density of the colloidal silica (which mean that a high proportion of air is comprised), together with the dilution effect of the additional pharmaceutically acceptable excipient, thereby effectively alleviating the defect that the pyrroloquinoline quinone trilithium salt nonahydrate compound is prone to form a rigid thin sheets which are not easy to granulate in dry granulation. The thin sheets as prepared by dry granulation are loose and easy to granulate, and thus the production efficiency and yield are improved, making it possible for commercial production scale-up. Meanwhile, due to the large specific surface area and good anti-adhesion effect, the colloidal silica can solve the problems of easy aggregation and adhesion to the surface of an equipment.

In addition, the pyrroloquinoline quinone trilithium salt nonahydrate capsule prepared by the composition and capsule preparation method therefor of the invention can make the crystalline form of the pyrroloquinoline quinone trilithium salt nonahydrate compound have good stability and dissolution profile. Experimental results show that the capsule can be subjected to long-term storage at a temperature of 30°C, and maintains a good dissolution profile at 25°C, 60% relative humidity and 30°C, 65% relative humidity after 6 months in both of the long-term and accelerated tests, no related substances are detected, indicating that the capsule comprising the pharmaceutical composition prepared by the process of the invention has good chemical stability. Moreover, due to the proper proportion of excipients used, excessive use of excipients is avoided, making the capsule as prepared suitable for elderly patients to swallow, convenient for elderly patients to take, thereby improving their medication compliance.

In order to make the above objects, features and advantages of the invention become more apparent, the invention will be described in detail in the following with reference to the accompanying drawings and specific embodiments.

Unless otherwise specified, the starting materials, samples, reagents, and equipment used in the examples of the invention are commercially available.

### 1. Starting materials

The API, pyrroloquinoline quinone trilithium salt nonahydrate crystal, can be prepared according to the preparation method in the Chinese invention patent granted text CN103351387B (application number: 201310270885.2, published on October 16, 2013), which will not be described in detail here. The pyrroloquinoline quinone trilithium salt nonahydrate compound prepared by the above preparation method has an X-ray powder diffraction pattern comprising characteristic diffraction peaks at 2θ diffraction angles of 6.2°±0.2°, 7.4°±0.2°, 7.9°±0.2° and 23.6°±0.2°.

### 2. Excipients

The specifications and supplier information of excipients used in Example 1 to Example 4 and Comparative Example 1 to Comparative Example 3 are as follows: colloidal silica, pregelatinized starch (Starch 1500, Colorcon), microcrystalline cellulose (VIVAPUR 101, JRS), magnesium stearate (MF2V, Liaoning Aoda), lactose (granulac200, MEGGLE), mannitol (160C, ROQUETTE), anhydrous calcium hydrogen phosphate (Fujicalin-SG, Fuji chemical), magnesium aluminum silicate (Neusilin UFL2, Fuji chemical), croscarmellose sodium (Ac-Di-Sol, FMC BioPolymer); copovidone (VA64 fine, BASF). Hollow capsule shell: hydroxypropylmethylcellulose hollow capsule (Vcaps Plus, Capsugel); gelatin hollow capsule (Coni-Snap, Capsugel).

Among them, colloidal silica, also known as aerosil or fumed silica, is usually prepared by the reaction of silicon tetrachloride in the flame of hydrogen and oxygen (Chinese Pharmacopoeia 2020, Part IV, Colloidal Silica, P719). The English names of colloidal silica include Colloidal Anhydrous Silica, Light Anhydrous Silicic Acid, Silica Colloidal Anhydrous, Colloidal Silicon Dioxide, or Fumed Silica Powder, etc. Commercially available products for pharmaceutical use include the Aerosil series from Evonik Degussa, HDK series from Wacker, CAB-O-SIL series from CABOT, as well as pharmaceutical grade colloidal silica series from Hubei Huifu Nanomaterial Co., Ltd. in China, usually with bulk density range of 0.02 to 0.05 g/cm³, and tap density range of 0.03 to 0.22 g/cm³. Colloidal silica, as a pharmaceutical excipient, is commonly used as a lubricant (glidant), adsorbent, stabilizer, stabilizer, and thickener. In an oral solid formulation, it is usually used as a glidant, at a conventional amount of 0.1% to 0.3% (Fang Liang, Pharmaceutics, 8th edition, People's Medical Publishing House, P127). As a lubricant (glidant), the amount employed ranges from 0.1% to 1.0% (Raymond C Rowe, Paul J Sheskey, Marian E Quinn ed., Handbook of Pharmaceutical Excipients, 6th edition, Pharmaceutical Press and the American Pharmacists Association, P186).

The above examples of the models and supplier information of the excipients and capsules are only intended to better describe the ratio range and effect of the examples, and the types and suppliers of the excipients and capsule shells of the invention are not limited thereto.

### 3. Equipment

TF-MINI dry granulator (manufacturer: Japan Freund Industrial); HD-5 multidirectional motion mixer (manufacturer: Shanghai Tianxiang Jiantai); Z25 capsule filling machine (manufacturer: Shandong Xinma); DPP80 automatic blister packaging machine (manufacturer: Jiangnan Pharmaceutical Machinery Factory). The above examples of equipment models and supplier information are only intended to better describe the implementation effect of the examples, and the equipment models and suppliers used in the preparation process of the invention are not limited thereto.

### Examples 1 to 4: Compositions, Capsules comprising pyrroloquinoline Quinone Trilithium Salt Nonahydrate composition and Preparation thereof

**Table 1 Formulation composition of Example 1**

| Composition formulation | | Content of each component in each capsule/mg | | | |
|---|---|---|---|---|---|
| | | Formulation No. 1 | Formulation No. 2 | Formulation No. 3 | Formulation No. 4 |
| Pyrroloquinoline Quinone Trilithium salt nonahydrate (I) | | 25.0 | 25.0 | 25.0 | 25.0 |
| Colloidal silica (II) | | 4.0 | 5.5 | 7.5 | 12.0 |
| Additional excipients | Pregelatinized starch | 100.0 | 75.0 | 50.0 | 100.0 |
| | Microcrystalline cellulose | 24.0 | 24.0 | 16.5 | 23.5 |
| | Magnesium stearate | 1.0 | 1.0 | 1.0 | 1.5 |
| | Total (III) | 125.0 | 100.0 | 67.5 | 125.0 |
| I: II: III | | 1: 0.16: 5.0 | 1: 0.22: 4.0 | 1: 0.30: 2.7 | 1: 0.48: 5.0 |

| | | | | | |
|---|---|---|---|---|---|
| Note: the contents prepared in Formulations No. 1, No. 2, No. 3 and No.4 were filled into hydroxypropylmethylcellulose hollow capsules No. 3, No. 3, No. 4 and No. 3, respectively. | | | | | |

**Table 2 Formulation composition of Example 2**

| Composition formulation | Content of each component in each capsule/mg | | | | |
|---|---|---|---|---|---|
| | Formulation No. 5 | Formulation No. 6 | Formulation No. 7 | Formulation No. 8 | |
| Pyrroloquinoline Quinone Trilithium salt nonahydrate (I) | 50.0 | 50.0 | 50.0 | 50.0 | |
| Colloidal silica (II) | | 8.0 | 11.0 | 15.0 | 24.0 |
| Additional excipients | Pregelatinized starch | 30.0 | 50.0 | 100.0 | 30.0 |
| | Microcrystalline cellulose | 19.0 | 29.0 | 33.5 | 19.0 |
| | Magnesium stearate | 1.0 | 1.0 | 1.5 | 1.0 |
| | Total (III) | 50.0 | 80.0 | 135.0 | 50.0 |
| I: II: III | | 1: 0.16: 1.0 | 1: 0.22: 1.6 | 1: 0.30: 2.7 | 1: 0.48: 1.0 |

| | | | | | |
|---|---|---|---|---|---|
| Note: the contents of Formulations No. 5, No. 6, No. 7 and No. 8 were filled into hydroxypropylmethylcellulose hollow capsules No. 4, No. 3, No. 3 and No.3, respectively. | | | | | |

**Table 3 Formulation composition of Example 3**

| Composition formulation | | Content of each component in each capsule/mg | | | |
|---|---|---|---|---|---|
| | | Formulation No. 9 | Formulation No. 10 | Formulation No. 11 | Formulation No. 12 |
| Pyrroloquinoline Quinone Trilithium salt nonahydrate (I) | | 5.0 | 20.0 | 75.0 | 100.0 |
| Colloidal silica (II) | | 2.0 | 8.0 | 15.0 | 20.0 |
| Additional excipients | Pregelatinized starch | 16.0 | 60.0 | 100.0 | 133.0 |
| | Microcrystalline cellulose | 4.0 | 19.0 | 49.0 | 65.0 |
| | Magnesium stearate | 0 | 1.0 | 1.0 | 2.0 |
| | Total (III) | 20.0 | 80.0 | 150.0 | 200.0 |
| I: II: III | | 1: 0.40: 4.0 | 1: 0.40: 4.0 | 1: 0.20: 2.0 | 1: 0.20: 2.0 |

| | | | | | |
|---|---|---|---|---|---|
| Note: the contents prepared in Formulations No. 9, No. 10 and No. 12 were filled into hydroxypropylmethylcellulose hollow capsules No. 4, No. 4 and No.2, respectively. The contents prepared in Formulation No. 11 were filled into gelatin hollow capsule No. 2. | | | | | |

**Table 4 Formulation composition of Example 4**

| Composition formulation | | Content of each component in each capsule/mg | | | |
|---|---|---|---|---|---|
| | | Formulation No. 13 | Formulation No. 14 | Formulation No. 15 | Formulation No. 16 |
| Pyrroloquinoline Quinone Trilithium salt nonahydrate (I) | | 25.0 | 25.0 | 50.0 | 50.0 |
| Colloidal silica | internal addition (IV) | 7.5 | 6.5 | 15.0 | 13.0 |
| | external addition | 0 | 1.0 | 0 | 2.0 |
| | Total (II) | 7.5 | 7.5 | 15.0 | 15.0 |
| | internal addition: external addition | -- | 6.5:1 | -- | 6.5:1 |
| Additional excipients | Starch (internal addition) | 22.0 | 22.0 | 0 | 0 |
| | Lactose (internal addition) | 0 | 0 | 86.0 | 86.0 |
| | Microcrystalline cellulose (internal addition) | 0 | 0 | 40.0 | 30.0 |
| | Microcrystalline cellulose (external addition) | 0 | 0 | 0 | 10.0 |
| | Mannitol (internal addition) | 44.0 | 44.0 | 0 | 0 |
| | Croscarmellose sodium (internal addition) | 0 | 0 | 6.0 | 4.0 |
| | Croscarmellose sodium (external addition) | 0 | 0 | 0 | 2.0 |
| | Copovidone (internal addition) | 0.5 | 0.5 | 0 | 0 |
| | Magnesium stearate (internal addition) | 1.0 | 0.7 | 3.0 | 2.0 |
| | Magnesium stearate (external addition) | 0 | 0.3 | 0 | 1.0 |
| | Total amount of internal addition (V) | 67.5 | 67.2 | 135.0 | 122.0 |
| | Total (III) | 67.5 | 67.5 | 135.0 | 135.0 |
| | internal addition: external addition | -- | 224:1 | -- | 9.38:1 |
| I: II: III | | 1: 0.3: 2.7 | 1: 0.3: 2.7 | 1: 0.3: 2.7 | 1: 0.3: 2.7 |
| I: IV: V | | 1: 0.30: 2.7 | 1: 0.26: 2.7 | 1: 0.30: 2.7 | 1: 0.26: 2.4 |

| | | | | | |
|---|---|---|---|---|---|
| Note: the contents prepared in Formulations No. 13, No. 14, No. 15 and No. 16 were filled into hydroxypropylmethylcellulose hollow capsules No .4, No. 4, No. 3 and No. 3, respectively. I: IV: V represents the ratio of internal addition amounts for dry granulation, which is used for the subsequent study of effect example 1. | | | | | |

### Preparation method:

The preparation methods of Example 1 (Formulations No. 1 to No. 4), Example 2 (Formulations No. 5 to No. 8), Example 3 (Formulations No. 9 to No. 12), and Example 4 (Formulations No. 13 and No. 15):
(1) starting material pretreatment: the pyrroloquinoline quinone trilithium salt nonahydrate compound API was subjected to 30-mesh sieve treatment, and the excipients were subjected to 40-mesh sieve treatment;
(2) starting material preparation: the pyrroloquinoline quinone trilithium salt nonahydrate compound API and excipients were weighed according to the formulation ratios;
(3) premixing: the pyrroloquinoline quinone trilithium salt nonahydrate compound API and all excipients were added into a multidirectional motion mixer, and mixed at a speed of 20 rpm for 15 min to ensure uniform mixing;
(4) dry granulation: the uniformly mixed pyrroloquinoline quinone trilithium salt nonahydrate compound API and all excipients were added into a dry granulator for dry granulation at a temperature of 15°C to 25°C and a relative humidity of 45% to 55%, granulation parameters: rolling pressure: 5.0 MPa ± 1.0 MPa, feeding speed: 15 rpm to 30 rpm, roller speed: 4 rpm to 8 rpm, and 20-mesh-granulation with the equipment's built-in device;
(5) intermediate determinations: a sample of the granules after dry granulation was taken, and intermediate testing for content and other items was performed;
(6) capsule filling: the actual filling amount was calculated based on the intermediate content and theoretical filling amount at a temperature of 15°C to 25°C and a relative humidity of 45% to 55%, and an appropriate capsule specification was selected; the qualified materials and hollow capsules were respectively placed in a powder tray and a capsule tray of a capsule filling machine, the capsules were filed at a speed of 18,000 capsules/hour, and the average loading difference of the capsules was controlled to be ≤ ± 5.0% and single loading difference was controlled to be ≤ ± 7.5%;
(7) packaging: in a DPP80 automatic blister packaging machine, the filled capsules were packaged in a double aluminum blister package at a speed of 1,200 plates/hour; and
(8) storing: the packaged capsule comprising pyrroloquinoline quinone trilithium salt nonahydrate was stored at room temperature (lower than 30°C).

Preparation method of Example 4 (Formulations No. 14 and No.16)
(1) starting material pretreatment: the pyrroloquinoline quinone trilithium salt nonahydrate compound API was subjected to 30-mesh sieve treatment, and the excipients were subjected to 40-mesh sieve treatment;
(2) starting material preparation: the pyrroloquinoline quinone trilithium salt nonahydrate compound API and the excipients including excipients for internal addition and excipients for external addition were weighed according to the formulation ratios;
(3) premixing: the pyrroloquinoline quinone trilithium salt nonahydrate compound API and the excipients for internal addition were added into a multidirectional motion mixer, and mixed at a speed of 20 rpm for 15 min to ensure uniform mixing;
(4) dry granulation: the uniformly mixed pyrroloquinoline quinone trilithium salt nonahydrate compound API and the excipients for internal addition were added into a dry granulator for dry granulation at a temperature of 15°C to 25°C and a relative humidity of 45% to 55%, granulation parameters: rolling pressure: 5.0 MPa ± 1.0 Mpa, feeding speed: 15 rpm to 30 rpm, roller speed: 4 rpm to 8 rpm, and 20-mesh-granulation with the equipment's built-in device;
(5) total mixing: the granules obtained after dry granulation and the excipients for external addition were added into a multidirectional motion mixer, and mixed at a speed of 20 rpm for 15 min;
(6) intermediate determination: a sample of the granules after total mixing was taken, and intermediate testing for content and other items was performed;
(7) capsule filling: the actual filling amount was calculated based on the intermediate content and theoretical filling amount at a temperature of 15°C to 25°C and a relative humidity of 45% to 55%, and an appropriate capsule specification was selected; the qualified materials and hollow capsules were respectively placed in a powder tray and a capsule tray of a capsule filling machine, the capsules were filed at a speed of 18,000 capsules/hour, and the average loading difference of the capsules was controlled to be ≤ ± 5.0% and single loading difference was controlled to be ≤ ± 7.5%;
(8) packaging: in a DPP80 automatic blister packaging machine, the filled capsules were packaged in double aluminum blister packages at a speed of 1,200 plates/hour; and
(9) storing: the packaged capsule comprising pyrroloquinoline quinone trilithium salt nonahydrate was stored at room temperature (lower than 30°C).

### Comparative Examples 1 to 3

**Table 5 Formulation composition of Comparative Example 1**

| Composition formulation | | Content of each component in each capsule/mg | |
|---|---|---|---|
| | | Formulation No. 1 | Formulation No. 2 |
| Pyrroloquinoline Quinone Trilithium salt nonahydrate (I) | | 25.0 | 50.0 |
| Colloidal silica (II) | | 7.5 | 0 |
| Aluminum magnesium silicate (IV) | | 0 | 15.0 |
| Additional excipients | Pregelatinized starch | 0 | 100.0 |
| | Microcrystalline cellulose | 16.5 | 33.5 |
| | Anhydrous calcium hydrogen phosphate | 50.0 | 0 |
| | Magnesium stearate | 1.0 | 1.5 |
| | Total (III) | 67.5 | 135.0 |
| (I+IV): II: III | | 1: 0.30: 2.7 | 1: 0: 2.1 |
| (I+V): II: (III-V) | | 1: 0.10: 0.2 | 1: 0: 2.7 |
| I: IV: III | | - | 1: 0.30: 2.7 |

| | | | |
|---|---|---|---|
| Note: the contents prepared in Formulations No. 1 and No. 2 were filled into hydroxypropylmethylcellulose hollow capsules No. 4 and No. 2, respectively. | | | |

**Table 6 Formulation composition of Comparative Example 2**

| Composition formulation | | Content of each component in each capsule/mg | | | |
|---|---|---|---|---|---|
| | | Formulation No. 3 | Formulation No. 4 | Formulation No. 5 | Formulation No. 6 |
| Pyrroloquinoline Quinone Trilithium salt nonahydrate (I) | | 25.0 | 50.0 | 25.0 | 50.0 |
| Colloidal silica (II) | | 0.3 | 2.0 | 13.0 | 30.0 |
| Additional excipients | Pregelatinized starch | 50.0 | 100.0 | 50.0 | 100.0 |
| | Microcrystalline cellulose | 16.5 | 33.0 | 16.5 | 33.0 |
| | Magnesium stearate | 1.0 | 2.0 | 1.0 | 2.0 |
| | Total (III) | 67.5 | 135.0 | 67.5 | 135.0 |
| I: II: III | | 1: 0.01: 2.7 | 1: 0.04: 2.7 | 1: 0.52: 2.7 | 1: 0.60: 2.7 |

| | | | | | |
|---|---|---|---|---|---|
| Note: the contents prepared in Formulations No. 3, No. 4, No. 5, and No. 6 were filled into hydroxypropylmethylcellulose hollow capsules No. 4, No. 3, No. 4 and No. 2, respectively. | | | | | |

**Table 7 Formulation composition of Comparative Example 3**

| Composition formulation | | Content of each component in each capsule/mg | | | |
|---|---|---|---|---|---|
| | | Formulation No. 7 | Formulation No. 8 | Formulation No. 9 | Formulation No. 10 |
| Pyrroloquinoline Quinone Trilithium salt nonahydrate (I) | | 50.0 | 50.0 | 50.0 | 50.0 |
| Colloidal silica (II) | | 8.0 | 8.0 | 8.0 | 8.0 |
| Additional excipients | Pregelatinized starch | 30.0 | 23.0 | 178.0 | 267.0 |
| | Microcrystalline cellulose | 9.6 | 6.7 | 119.0 | 178.5 |
| | Magnesium stearate | 0.4 | 0.3 | 3.0 | 4.5 |
| | Total (III) | 40.0 | 30.0 | 300.0 | 450.0 |
| I: II: III | | 1: 0.16: 0.8 | 1: 0.16: 0.6 | 1: 0.16: 6.0 | 1: 0.16: 9.0 |

| | | | | | |
|---|---|---|---|---|---|
| Note: the contents of Formulations No. 7, No. 8, No. 9 and No.10 were filled into hydroxypropylmethylcellulose hollow capsules No. 4, No. 4, No. 1 and No. 0, respectively. | | | | | |

### Preparation methods:

The preparation methods of Comparative Example 1 (Formulations No. 1 and No. 2), Comparative Example 2 (Formulations No. 3 to No. 6), and Comparative Example 3 (Formulations No. 7 to No. 10) were the same as that in Example 1.

### Comparison of preparation results of Examples 1 to 4 with Comparative Examples 1 to 3

By comparing dry granulation of Formulation No. 5 of Comparative Example 2 with Formulation No. 6 of Comparative Example 2, it was found that due to the high proportion of colloidal silica added, the obtained thin sheets were too soft and prone to material adhesion on the surface of the roller shaft, making it impossible to achieve continuous pressing. However, during the dry granulation process, no obvious adhesion phenomenon on the surface of the roller shaft was observed in the formulations of Examples 1 to 4 and the remaining formulations of Comparative Examples 1 to 3.

The difference in the subsequent granulation results after dry rolling into thin sheets is shown in Effect Example 1.

In Examples 1 to 4 and Comparative Examples 1 to 3, except for the dry granulation step, no abnormalities or issues affecting subsequent production scale-up or stability were found in the remaining preparation process steps of pyrroloquinoline quinone trilithium salt nonahydrate capsules.

The following will further illustrate the advantages of the capsule comprising pyrroloquinoline quinone trilithium salt nonahydrate and the preparation method of the invention by performing performance tests on the final products of Examples 1 to 4 and Comparative Examples 1 to 3.

### Effect Example 1

In order to compare and demonstrate the effect of colloidal silica on improving granulation of pyrroloquinoline quinone trilithium salt nonahydrate, which are prone to compression into hard thin sheets, 20 g ± 2 g of thin sheets (thickness between 0.8 mm and 1.0 mm) of pyrroloquinoline quinone trilithium salt nonahydrate after dry granulation prepared in Examples 1 to 4 and Comparative Examples 1 and 3 was each taken and placed into a granulation equipment built in the dry granulation equipment, and passed through a 20-mesh 316L stainless steel screen at a fixed speed (96 rpm, motor power: 0.2KW, unidirectional rotary granulation) set by the equipment. The granulation time was fixed at 15 min. After granulation, the materials remained on the screen were collected, weighed, and denoted as A (g), and the materials passing through the screen were collected, weighed, and denoted as B (g). The granulation yield was calculated according to W = B/ (A + B) × 100%.

**Table 8 Granulation yield of Examples 1 to 4**

| Composition formulation | Example 1 | | | |
|---|---|---|---|---|
| | Formulation No. 1 | Formulation No. 2 | Formulation No. 3 | Formulation No. 4 |
| Granulation yield (%) | 100.0% | 100.0% | 100.0% | 100.1% |

| Composition formulation | Example 2 | | | |
|---|---|---|---|---|
| | Formulation No. 5 | Formulation No. 6 | Formulation No. 7 | Formulation No. 8 |
| Granulation yield (%) | 91.1% | 96.9% | 99.5% | 96.7% |

| Composition formulation | Example 3 | | | |
|---|---|---|---|---|
| | Formulation No. 9 | Formulation No. 10 | Formulation No. 11 | Formulation No. 12 |
| Granulation yield (%) | 98.8% | 98.8% | 99.5% | 97.3% |

| Composition formulation | Example 4 | | | |
|---|---|---|---|---|
| | Formulation No. 13 | Formulation No. 14 | Formulation No. 15 | Formulation No. 16 |
| Granulation yield (%) | 99.9% | 99.9% | 99.8% | 100.0% |

**Table 9 Granulation yield of Comparative Examples 1 to 3**

| Composition formulation | Comparative Example 1 | | | |
|---|---|---|---|---|
| | Formulation No. 1 | | Formulation No. 2 | |
| Granulation yield (%) | 46.1% | | 50.1% | |

| Composition formulation | Comparative Example 2 | | | |
|---|---|---|---|---|
| | Formulation No. 3 | Formulation No. 4 | Formulation No. 5 | Formulation No. 6 |
| Granulation yield (%) | 64.1% | 67.7% | 100.0% | 100.0% |

| Composition formulation | Comparative Example 3 | | | |
|---|---|---|---|---|
| | Formulation No. 7 | Formulation No. 8 | Formulation No. 9 | Formulation No. 10 |
| Granulation yield (%) | 37.2% | 35.8% | 99.2% | 100.0% |

Granulation results: according to the results in Table 8 and Table 9, the granulation yield of the capsules prepared with the Formulations No.1 to No. 16 of Examples 1 to 4 was always greater than 90%. The colloidal silica is preferably 0.2 part by weight to 0.4 part by weight, the additional pharmaceutically acceptable excipients are preferably 2.0 parts by weight to 4.0 parts by weight, and the granulation yield of capsules with Formulations No. 2 and No. 3 in Example 1, Formulation No. 7 in Example 2, Formulations No. 9 to No. 12 in Example 3 and Formulations No. 13 to No. 16 in Example 4 was always greater than 97%.

In Comparative Example 1, although the capsule with Formulation No. 1 contained colloidal silica, the addition of a certain proportion of other metal salts such as anhydrous calcium hydrogen phosphate made the material hard and difficult to process, and the yield was only 46.1% after granulating for 15 min. In Formulation No.2 of Comparative Example 1, the colloidal silica of Formulation No. 7 of Example 2 was replaced by another metal salt, UFL2 type magnesium aluminum silicate, which also has an extremely high specific surface area (300 m²/g), a low bulk density (0.06 to 0.11 g/cm³), and a tap density (0.10 to 0.17 g/cm³), as a result, the granulation yield decreased from 99.5% to 50.1%. The reason for the above difference is that the metal salt aluminum magnesium silicate could increase the rigidity of granules, while colloidal silica could resist or reduce rigidity.

In Comparative Example 2, the ratio of pyrroloquinoline quinone trilithium salt nonahydrate and the additional pharmaceutically acceptable excipients were fixed at 1: 2.7, and the proportion of colloidal silica was changed to be lower than 0.16. As shown by the results of Formulations No. 3 and No. 4, the yield after granulation was only 64.1% and 67.7%, respectively. When the proportion of colloidal silica was increased to greater than 0.48, as shown by Formulations No. 5 and No. 6, the material became loose and easy to be granulated, and the yield reached 100.0%. However, due to the large proportion of colloidal silica, the resulting thin sheets were too soft, and serious roller adhesion was observed in the dry granulation process.

In Comparative Example 3, the ratio of pyrroloquinoline quinone trilithium salt nonahydrate and colloidal silica was fixed at 1: 0.16, and the proportion of the additional pharmaceutically acceptable excipients was changed to less than 1.0, as shown by Formulations No. 7 and No. 8, the yield after granulation was only 37.2% and 35.8%, respectively, indicating that in addition to colloidal silica, it is also important to maintain a certain proportion of the additional pharmaceutically acceptable excipients. When the proportion of the additional pharmaceutically acceptable excipients was increased to higher than 5.0, as shown by Formulations No. 9 and No. 10, the materials became loose and easy to be granulated, and the yield was greater than 99.0%; but with the increase of the proportion of the additional pharmaceutically acceptable excipients, the proportion of excipients in the content needs to be greatly increased accordingly, and the capsule volume size has to be expanded for a drug of the same specification. For example, Formulations No. 9 and No. 10 of Comparative Example 3, due to the large amount of materials, had to be filled into capsules No. 1 and No. 0, the long diameters of which are 18.5 to 19.5 mm and 20.7 to 21.7 mm, respectively; while Formulations No. 5 to No. 8 of Example 2 at a similar specification (50 mg) were filled into capsules No. 4 and No. 3, the long diameters of which are 13.4 to 14.3 mm and 15.0 to 15.8 mm, respectively. The variations in the capsule size as described above increases the difficulty of swallowing for elderly patients.

### Effect Example 2

Pyrroloquinoline quinone trilithium salt nonahydrate capsules prepared in Examples 1 to 4 and Comparative Examples 1 to 3 were taken (6 capsules for each formulation) to determine the dissolution profiles by the method described below.

Dissolution test conditions: with 900 mL of a hydrochloric acid solution (37 ± 0.2°C, pH 1.2) as the dissolution medium, and at a rotate speed of 50 rpm, the capsule was placed in a settling basket and then placed into a dissolution cup; samples were taken at predetermined time points, filtered with 0.45 µm microporous membrane made of mixed cellulose ester, and the filtrate was used as the test solution.

Dissolution determination method: octadecylsilane bonded silica gel was used as filler (Welch Ultimate^{®} XB-C₁₈ 4.6*150mm, 5 µm or an equivalent chromatographic column), 10 mM dipotassium hydrogen phosphate-15 mM tetrabutylammonium bromide (adjusted to pH 6.8 with phosphoric acid): acetonitrile (65:35) was used as the mobile phase, flow rate was 1.0 mL/min, column temperature was 30°C, and detection wavelength was 250 nm. 20 µL of a reference solution and 20 µL of the test solution were precisely taken, and injected into liquid chromatograph, respectively; the chromatogram was recorded, the concentration at each time point was calculated by peak area according to an external standard method, and the cumulative dissolution percentage at each time point was calculated.

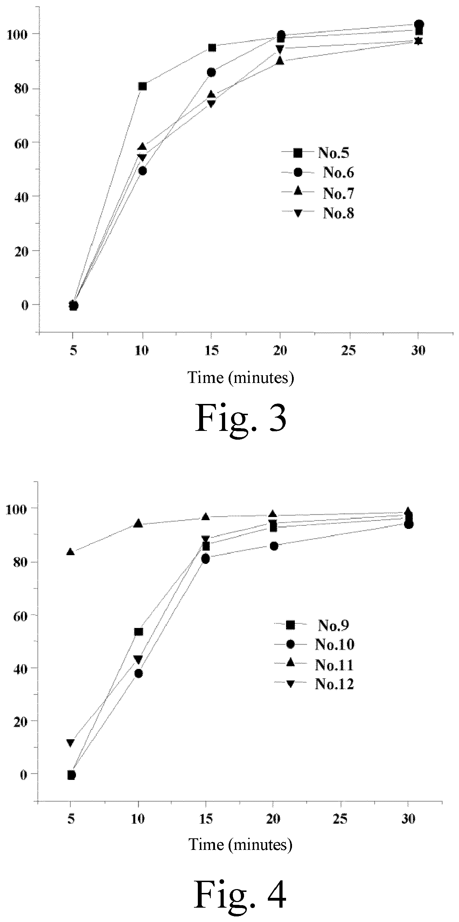

Dissolution results: according to the results in FIG 2 to 5 and Tables 10 to 13, the capsules Formulations No. 1 to No. 16 prepared according to Example 1 to Example 4 achieved rapid dissolution with the cumulative dissolution percentage Q greater than 80% after 20 min and the cumulative dissolution percentage Q greater than 90% after 30 min.

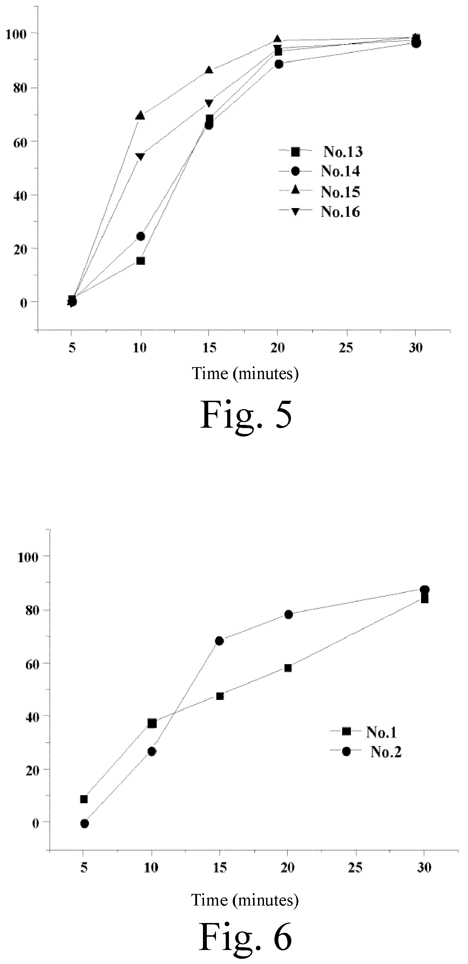

According to the results in Table 14 and FIG 6, in Formulation No. 1 of Comparative Example 1, the additional pharmaceutically acceptable excipients of the composition comprised a certain proportion of the other metal salt (anhydrous calcium phosphate), it had an increased hardness after dry granulation, and the dissolution rate of the resulting capsules was decreased, with cumulative dissolution at 20 min and 30 min decreased to 58.3% and 84.5%, respectively. In Formulation No.2 of Comparative Example 1, the colloidal silica was replaced with UFL2 magnesium aluminum silicate, which also has an extremely high specific surface area (300 m²/g), a low bulk density (0.06 to 0.11 g/cm³), and a tap density (0.10 to 0.17 g/cm³), as a result, the cumulative dissolution of the capsules as prepared decreased to 78.3% and 87.7% after 20 min and 30 min, respectively.

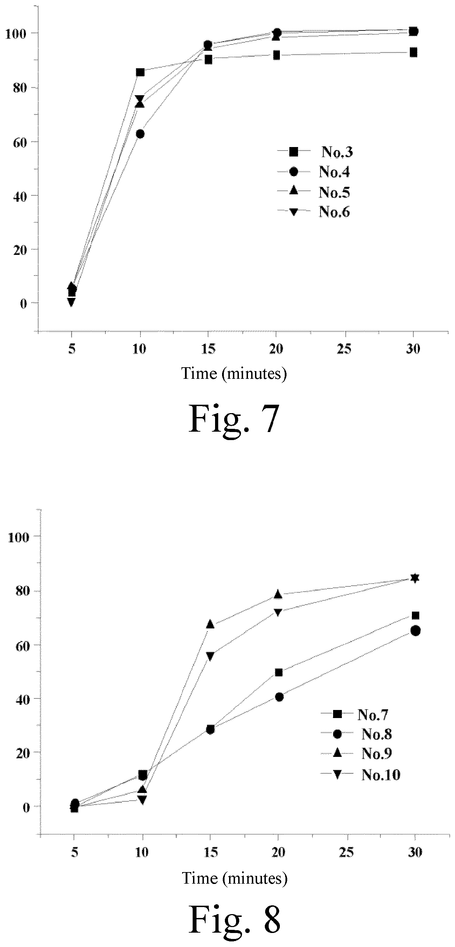

According to the results in Table 15 and FIG 7, in Comparative Example 2, when the weight ratio of the additional external excipients was fixed at 2.7, Formulations No. 3 to No. 6 containing colloidal silica at a low or high ratio achieved cumulative dissolution of greater than 80% after 20 min and greater than 90% after 30 min, and even achieved complete dissolution. However, in terms of the process, the material was hard and difficult to crush at a low ratio of colloidal silica (see Table 9 in Effect Example 1), and the material stuck to the surface of the roller at a high ratio of colloidal silica (see the description of preparation results in Comparative Example 2).

According to the results in Table 16 and FIG 8, in Comparative Example 3, when 0.16 times of colloidal silica was added, at both a low proportion and a high proportion of the additional excipients, the cumulative dissolution could not exceed 80% after 20 min and could not exceed 90% after 30 min.

**Table 10 Dissolution Results of Capsules of Example 1 (n = 6)**

| Time (min) | Cumulative dissolution percentage Q (%) | | | |
|---|---|---|---|---|
| | Formulation No. 1 | Formulation No. 2 | Formulation No. 3 | Formulation No. 4 |
| 5 | 18.1 | 1.0 | 0.3 | 15.9 |
| 10 | 64.9 | 80.9 | 63.6 | 78.6 |
| 15 | 83.5 | 98.5 | 71.4 | 94.5 |
| 20 | 96.5 | 101.6 | 84.2 | 97.5 |
| 30 | 102.3 | 102.5 | 97.4 | 98.4 |

**Table 11 Dissolution Results of Capsules of Example 2 (n = 6)**

| Time (min) | Cumulative dissolution percentage Q (%) | | | |
|---|---|---|---|---|
| | Formulation No. 5 | Formulation No. 6 | Formulation No. 7 | Formulation No. 8 |
| 5 | 0.0 | 0.0 | 0.0 | 0.0 |
| 10 | 80.9 | 49.4 | 58.0 | 54.6 |
| 15 | 95.2 | 86.1 | 77.3 | 74.6 |
| 20 | 98.6 | 99.6 | 89.7 | 94.5 |
| 30 | 101.3 | 103.8 | 97.2 | 97.6 |

**Table 12 Dissolution Results of Capsules of Example 3 (n = 6)**

| Time (min) | Cumulative dissolution percentage Q (%) | | | |
|---|---|---|---|---|
| | Formulation No. 9 | Formulation No. 10 | Formulation No. 11 | Formulation No. 12 |
| 5 | 0.0 | 0.0 | 83.2 | 11.9 |
| 10 | 53.8 | 37.9 | 94.1 | 43.2 |
| 15 | 86.2 | 81.4 | 96.5 | 88.7 |
| 20 | 93.1 | 86.1 | 97.6 | 94.6 |
| 30 | 96.4 | 94.2 | 98.7 | 97.5 |

**Table 13 Dissolution Results of Capsules of Example 4 (n = 6)**

| Time (min) | Cumulative dissolution percentage Q (%) | | | |
|---|---|---|---|---|
| | Formulation No.13 | Formulation No.14 | Formulation No.15 | Formulation No.16 |
| 5 | 1.2 | 0.5 | 0.0 | 0.0 |
| 10 | 15.6 | 24.6 | 69.2 | 54.6 |
| 15 | 68.3 | 66.4 | 86.3 | 74.6 |
| 20 | 93.5 | 88.9 | 97.6 | 94.5 |
| 30 | 98.6 | 96.8 | 98.4 | 97.6 |

**Table 14 Dissolution Results of Capsules of Comparative Example 1 (n = 6)**

| Time (min) | Cumulative dissolution percentage Q (%) | |
|---|---|---|
| | Formulation No. 1 | Formulation No. 2 |
| 5 | 9.0 | 0.0 |
| 10 | 37.5 | 27.0 |
| 15 | 47.8 | 68.6 |
| 20 | 58.3 | 78.3 |
| 30 | 84.5 | 87.7 |

**Table 15 Dissolution Results of Capsules of Comparative Example 2 (n = 6)**

| Time (min) | Cumulative dissolution percentage Q (%) | | | |
|---|---|---|---|---|
| | Formulation No. 3 | Formulation No. 4 | Formulation No. 5 | Formulation No. 6 |
| 5 | 4.5 | 5.6 | 6.2 | 0.7 |
| 10 | 85.9 | 63.1 | 73.5 | 76.0 |
| 15 | 90.4 | 95.9 | 94.4 | 96.1 |
| 20 | 92.1 | 100.5 | 98.6 | 99.8 |
| 30 | 93.1 | 101.0 | 100.0 | 101.2 |

**Table 16 Dissolution Results of Capsules of Comparative Example 3 (n = 6)**

| Time (min) | Cumulative dissolution percentage Q (%) | | | |
|---|---|---|---|---|
| | Formulation No. 7 | Formulation No. 8 | Formulation No. 9 | Formulation No. 10 |
| 5 | 0.0 | 1.3 | 0.0 | 0.0 |
| 10 | 12.0 | 11.7 | 6.2 | 2.9 |
| 15 | 29.0 | 28.9 | 67.0 | 56.0 |
| 20 | 49.9 | 41.0 | 78.2 | 72.3 |
| 30 | 70.9 | 65.4 | 84.6 | 84.7 |

### Effect example 3

The capsules prepared according to Formulation No. 3 in Example 1 and Formulation No. 7 in Example 2, packaged in double aluminum blisters, were tested at 25°C, 60% relative humidity and 30°C, 65% relative humidity for long term and accelerated tests for 6 months. The dissolution profiles, related substances, and PXRD of the samples before and after the tests were determined.

### Dissolution test conditions: the same as that in Effect Example 2

Determination method of related substances: octadecylsilane bonded silica was used as filler (TCI Kaseisorb LC ODS 2000, 4.6*150mm or chromatographic column with equivalent efficiency); 10 mM dipotassium hydrogen phosphate-15 mM tetrabutylammonium bromide buffer solution (preparation method: 2.28 g of dipotassium hydrogen phosphate trihydrate and 4.84 g of tetrabutylammonium bromide were taken, diluted with 1 L water, and adjusted to a pH value of 7.4 with phosphoric acid) was used as mobile phase A and acetonitrile was used as mobile phase B, and gradient elution was performed according to Table 9; flow rate: 1.0 mL/min, detection wavelength: 250 nm, and column temperature: 30°C.

**Table 17 Gradient elution conditions of liquid chromatography**

| Time (min) | Mobile phase A (%) | Mobile phase B (%) |
|---|---|---|
| 0.0 | 70 | 30 |
| 35.0 | 70 | 30 |
| 45.0 | 35 | 65 |
| 50.0 | 35 | 65 |
| 50.01 | 70 | 30 |
| 55.0 | 70 | 30 |

The content of the capsule was accurately weighed, ultrasonically diluted with a 30% acetonitrile aqueous solution to prepare a solution containing about 0.2 mg of pyrroloquinoline quinone trilithium salt nonahydrate per 1 mL as the test solution. The test solution was diluted by 100 folds with 30% aqueous acetonitrile as a self-control solution. 20 µL of the test solution and 20 µL of the self-control solution were precisely injected into liquid chromatograph, respectively, and chromatograms were recorded. The related substances of pyrroloquinoline quinone trilithium salt nonahydrate were calculated by a self-control method. 0.01 time of the peak area of the self-control solution was taken as the reporting threshold of an individual impurity.

PXRD determination method: it was tested according to General Rule 0451, Part IV, Chinese Pharmacopoeia, Edition 2015; conditions: CuKa40Kv 40mA, emission slit: 1.0 mm, Soller slit: 0.4°, continuous scanning, step size: 0.02°, speed: 8°/min, detector: LynxEye. Instrument model: Bruker D8 ADVANCE.

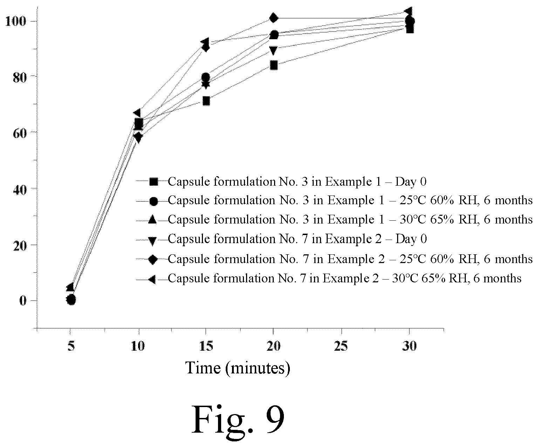

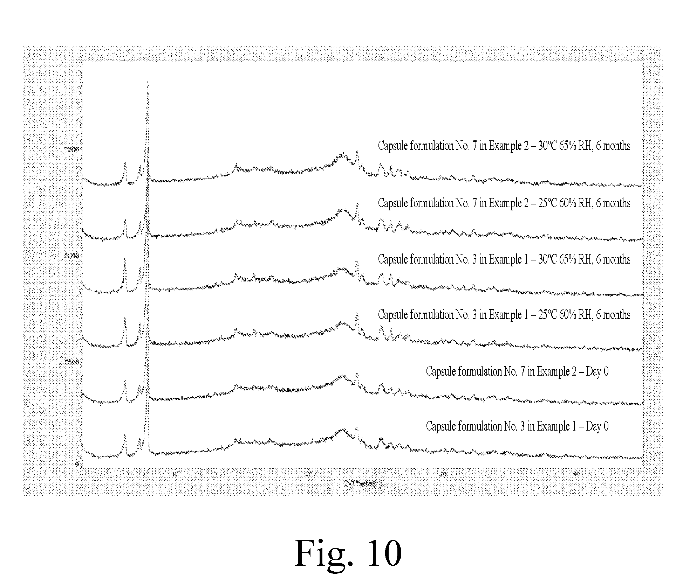

Results: the determination results of the dissolution profile are shown in Table 18 and FIG 9. After long-term and accelerated tests for 6 months at 25°C, 60% relative humidity and 30°C, 65% relative humidity (RH), the dissolution results of the capsules prepared with Formulation No. 3 of Example 1 and Formulation No. 7 of Example 2 showed no significant changes, maintaining cumulative dissolution greater than 80% after 20 min and greater than 90% after 30 min. In addition, no related substances were detected in the above capsule samples before or after the stability test, indicating that the capsules containing pyrroloquinoline quinone trilithium salt nonahydrate prepared by the process of the invention have good chemical stability. The results of PXRD are shown in FIG 10. According to these results, the characteristic diffraction peak intensity and geometric topology of the capsule contents containing pyrroloquinoline quinone trilithium salt nonahydrate after long-term and accelerated tests for 6 months were kept consistent with those on Day 0, indicating that the pyrroloquinoline quinone trilithium salt nonahydrate in the capsules has good crystal stability.

**Table 18 Dissolution profile results of pyrroloquinoline quinone trilithium salt nonahydrate capsules (n = 6)**

| Time (min) | Cumulative dissolution percentage Q (%) | | | | | |
|---|---|---|---|---|---|---|
| | Formulation No. 3 of Example 1 | | | Formulation No. 7 of Example 2 | | |
| | Day 0 | Long-term 6 months | Accelerated 6 months | Day 0 | Long-term 6 months | Accelerated 6 months |
| 5 | 0.3 | 0.3 | 3.7 | 0.0 | 0.0 | 4.6 |
| 10 | 63.6 | 63.7 | 61.4 | 58.0 | 58.5 | 66.9 |
| 15 | 71.4 | 80.1 | 77.3 | 77.3 | 90.5 | 92.3 |
| 20 | 84.2 | 95.3 | 94.2 | 89.7 | 101.1 | 95.2 |
| 30 | 97.4 | 100.1 | 98.6 | 97.2 | 100.8 | 103.5 |

**Table 19 Determination results of related substances of pyrroloquinoline quinone trilithium salt nonahydrate capsules**

| | Related substances (%) | | | | | |
|---|---|---|---|---|---|---|
| Name | Formulation No. 3 of Example 1 | | | Formulation No. 7 of Example 2 | | |
| | Day 0 | 6 months | | Day 0 | 6 months | |
| | | Long term | Accelerate | | Long | Accelerate |
| | | | d | | term | d |
| Individual impurity | Not detected | Not detected | Not detected | Not detected | Not detected | Not detected |
| Total impurities | Not detected | Not detected | Not detected | Not detected | Not detected | Not detected |

Although the invention is disclosed as above, it is not limited thereto.

## Claims

1. A pharmaceutical composition comprising a pyrroloquinoline quinone trilithium salt nonahydrate compound, **characterized in that** the pharmaceutical composition comprises:
1.0 part by weight of pyrroloquinoline quinone trilithium salt nonahydrate compound; and a pharmaceutically acceptable excipient;
the pharmaceutically acceptable excipient comprises:
0.16 part by weight to 0.48 part by weight of colloidal silica;
wherein the pharmaceutically acceptable excipient further comprises:
1.0 part by weight to 5.0 parts by weight of additional pharmaceutically acceptable excipient(s);
the additional pharmaceutically acceptable excipient is selected from one or more of starch, pregelatinized starch, microcrystalline cellulose, lactose, lactose starch complex, lactose cellulose complex, mannitol, mannitol starch complex, sorbitol, povidone, copovidone, hydroxypropylmethylcellulose, hydroxypropylcellulose, low-substituted hydroxypropylcellulose, croscarmellose sodium, crospovidone, magnesium stearate, sodium stearyl fumarate, pulvis talci and stearic acid;
wherein the pyrroloquinoline quinone trilithium salt nonahydrate compound is in a crystalline form, and the pyrroloquinoline quinone trilithium salt nonahydrate compound has an X-ray powder diffraction pattern comprising characteristic diffraction peaks at 2θ diffraction angles of 6.2°±0.2°, 7.4°±0.2°, 7.9°±0.2° and 23.6°±0.2°.

2. The pharmaceutical composition comprising the pyrroloquinoline quinone trilithium salt nonahydrate compound according to claim 1, **characterized in that** the colloidal silica is of 0.2 part by weight to 0.4 part by weight; and the additional pharmaceutically acceptable excipient is of 2.0 parts by weight to 4.0 parts by weight.

3. The pharmaceutical composition comprising the pyrroloquinoline quinone trilithium salt nonahydrate compound according to claim 2, **characterized in that** the additional pharmaceutically acceptable excipient comprises: 0.6 part by weight to 4 parts by weight of pregelatinized starch; 0.36 part by weight to 0.96 part by weight of microcrystalline cellulose; and 0 part by weight to 0.06 part by weight of magnesium stearate.

4. The pharmaceutical composition comprising the pyrroloquinoline quinone trilithium salt nonahydrate compound according to claim 1, **characterized in that** the colloidal silica can be added through an internal addition method or an internal-external addition method; and the additional pharmaceutically acceptable excipient can be added through an internal addition method or an internal-external addition method.

5. The pharmaceutical composition comprising the pyrroloquinoline quinone trilithium salt nonahydrate compound according to claim 4, **characterized in that** when the colloidal silica is added through the internal-external addition method, the weight ratio of the internally added colloidal silica to the externally added colloidal silica is greater than 3:1; and when the additional pharmaceutically acceptable excipient is added through the internal-external addition method, the weight ratio of the internally added additional pharmaceutically acceptable excipient to the externally added additional pharmaceutically acceptable excipient is greater than 3:1.

6. The pharmaceutical composition comprising the pyrroloquinoline quinone trilithium salt nonahydrate compound according to claim 4, **characterized in that** the additional pharmaceutically acceptable excipient which can be added through the internal addition method is selected from one or more of starch, lactose, microcrystalline cellulose, mannitol, croscarmellose sodium, copovidone, and magnesium stearate; and the additional pharmaceutically acceptable excipient which can be added through the external addition method is selected from one or more of microcrystalline cellulose, croscarmellose sodium, and magnesium stearate.

7. A capsule comprising a pyrroloquinoline quinone trilithium salt nonahydrate compound, **characterized in that** the capsule comprising the pyrroloquinoline quinone trilithium salt nonahydrate compound comprises the pharmaceutical composition according to any one of claims 1-6.

8. The capsule comprising the pyrroloquinoline quinone trilithium salt nonahydrate compound according to claim 7, **characterized in that** the capsule material can be selected from any one of gelatin capsule, hydroxypropylmethylcellulose capsule, pullulan polysaccharide capsule, and starch capsule.

9. The capsule comprising the pyrroloquinoline quinone trilithium salt nonahydrate compound according to claim 8, **characterized in that** the capsule material is hydroxypropylmethylcellulose capsule.

10. The capsule comprising the pyrroloquinoline quinone trilithium salt nonahydrate compound according to claim 7, **characterized in that** in the capsule formulation, each capsule comprises 5 mg to 100 mg of pyrroloquinoline quinone trilithium salt nonahydrate compound.

11. The capsule comprising the pyrroloquinoline quinone trilithium salt nonahydrate compound according to claim 10, **characterized in that** each capsule comprises 20 mg to 75 mg of pyrroloquinoline quinone trilithium salt nonahydrate compound.

12. A method for preparing a capsule comprising the pyrroloquinoline quinone trilithium salt nonahydrate compound, which is employed for preparing the capsule comprising the pyrroloquinoline quinone trilithium salt nonahydrate compound according to claim 7, **characterized in that** the preparation method is a dry granulation method for preparing a capsule.

13. The method for preparing the capsule comprising the pyrroloquinoline quinone trilithium salt nonahydrate compound according to claim 12, **characterized in that** when the pharmaceutically acceptable excipient is added through the internal addition method, the preparation method comprises:
weighing the active pharmaceutical ingredient (API) of the pyrroloquinoline quinone trilithium salt nonahydrate compound and the pharmaceutically acceptable excipient according to the formulation ratios;
mixing the pyrroloquinoline quinone trilithium salt nonahydrate compound API with the pharmaceutically acceptable excipient;
performing dry granulation on the mixed pyrroloquinoline quinone trilithium salt nonahydrate compound API and the pharmaceutically acceptable excipient, firstly rolling the mixture into thin sheets, and then granulating into granules; and
performing capsule filling with the granules to obtain the capsule.

14. The method for preparing the capsule comprising the pyrroloquinoline quinone trilithium salt nonahydrate compound according to claim 12, **characterized in that** when the pharmaceutically acceptable excipient is added through the internal-external addition method, the preparation method comprises:
weighing the active pharmaceutical ingredient (API) of the pyrroloquinoline quinone trilithium salt nonahydrate compound and the pharmaceutically acceptable excipient according to the formulation ratios;
mixing the pyrroloquinoline quinone trilithium salt nonahydrate compound API and the pharmaceutically acceptable excipient for internal addition;
performing dry granulation on the mixed pyrroloquinoline quinone trilithium salt nonahydrate compound API and the pharmaceutically acceptable excipient for internal addition, firstly rolling the mixture into thin sheets, and then granulating into granules;
mixing the granules obtained by the dry granulation with the pharmaceutically acceptable excipient for external addition; and
performing capsule filling with the granules mixed with the pharmaceutically acceptable excipient for external addition, to obtain the capsule.

## Patentansprüche

1. Pharmazeutische Zusammensetzung, die eine Pyrrolochinolinchinon-Trilithiumsalz-Nonahydrat-Verbindung umfasst, **gekennzeichnet dadurch, dass** die pharmazeutische Zusammensetzung Folgendes umfasst:
1,0 Gew.-Teil Pyrrolochinolinchinon-Trilithiumsalz-Nonahydrat-Verbindung; und einen pharmazeutisch akzeptablen Hilfsstoff;
wobei der pharmazeutisch akzeptable Hilfsstoff Folgendes umfasst:
0,16 Gew.-Teile bis 0,48 Gew.-Teile kolloidales Siliziumdioxid;
wobei der pharmazeutisch akzeptable Hilfsstoff ferner Folgendes umfasst:
1,0 Gew.-Teil bis 5,0 Gew.-Teile eines zusätzlichen/zusätzlicher pharmazeutisch akzeptablen/akzeptabler Hilfsstoffs/Hilfsstoffe;
wobei der zusätzliche pharmazeutisch akzeptable Hilfsstoff aus Stärke, vorverkleisterter Stärke, mikrokristalliner Cellulose, Laktose, einem Laktose-Stärke-Komplex, einem Laktose-Cellulose-Komplex, Mannitol, einem Mannitol-Stärke-Komplex, Sorbitol, Povidon, Copovidon, Hydroxypropylmethylcellulose, Hydroxypropylcellulose, niedrigsubstituierter Hydroxypropylcellulose, Croscarmellose-Natrium, Crospovidon, Magnesiumstearat, Natriumstearylfumarat, Talkumpuder und/oder Stearinsäure ausgewählt ist;
wobei die Pyrrolochinolinchinon-Trilithiumsalz-Nonahydrat-Verbindung in kristalliner Form vorliegt und die Pyrrolochinolinchinon-Trilithiumsalz-Nonahydrat-Verbindung ein Röntgenpulverdiffraktionsmuster aufweist, das charakteristische Diffraktionspeaks bei 2θ-Diffraktionswinkeln von 6,2°±0,2°, 7,4°±0,2°, 7,9°±0,2° und 23,6°±0,2° aufweist.

2. Pharmazeutische Zusammensetzung, die die Pyrrolochinolinchinon-Trilithiumsalz-Nonahydrat-Verbindung nach Anspruch 1 umfasst, **gekennzeichnet dadurch, dass** das kolloidale Siliziumdioxid 0,2 Gew.-Teile bis 0,4 Gew.-Teile beträgt; und der zusätzliche pharmazeutisch akzeptable Hilfsstoff 2,0 Gew.-Teile bis 4,0 Gew.-Teile beträgt.

3. Pharmazeutische Zusammensetzung, die die Pyrrolochinolinchinon-Trilithiumsalz-Nonahydrat-Verbindung nach Anspruch 2 umfasst, **gekennzeichnet dadurch, dass** der zusätzliche pharmazeutisch akzeptable Hilfsstoff Folgendes umfasst: 0,6 Gew.-Teile bis 4 Gew.-Teile vorverkleisterte Stärke; 0,36 Gew.-Teile bis 0,96 Gew.-Teile mikrokristalline Cellulose; und 0 Gew.-Teile bis 0,06 Gew.-Teile Magnesiumstearat.

4. Pharmazeutische Zusammensetzung, die die Pyrrolochinolinchinon-Trilithiumsalz-Nonahydrat-Verbindung nach Anspruch 1 umfasst, **dadurch gekennzeichnet** dadurch, dass das kolloidale Siliziumdioxid durch ein internes Zugabeverfahren oder ein internes-externes Zugabeverfahren zugegeben werden kann; und der zusätzliche pharmazeutisch akzeptable Hilfsstoff durch ein internes Zugabeverfahren oder ein internes-externes Zugabeverfahren zugegeben werden kann.

5. Pharmazeutische Zusammensetzung, die die Pyrrolochinolinchinon-Trilithiumsalz-Nonahydrat-Verbindung nach Anspruch 4 umfasst, **gekennzeichnet dadurch, dass**, wenn das kolloidale Siliziumdioxid durch das interne-externe Zugabeverfahren zugegeben wird, das Gewichtsverhältnis des intern zugegebenen kolloidalen Siliziumdioxids zu dem extern zugegebenen kolloidalen Siliziumdioxid größer als 3:1 ist; und dass, wenn der zusätzliche pharmazeutisch akzeptable Hilfsstoff durch das interne-externe Zugabeverfahren zugegeben wird, das Gewichtsverhältnis des intern zugegebenen zusätzlichen pharmazeutisch akzeptablen Hilfsstoffs zu dem extern zugegebenen zusätzlichen pharmazeutisch akzeptablen Hilfsstoff größer als 3:1 ist.

6. Pharmazeutische Zusammensetzung, die die Pyrrolochinolinchinon-Trilithiumsalz-Nonahydrat-Verbindung nach Anspruch 4 umfasst, **gekennzeichnet dadurch, dass** der zusätzliche pharmazeutisch akzeptable Hilfsstoff, der durch das interne Zugabeverfahren zugegeben werden kann, aus Stärke, Laktose, mikrokristalliner Cellulose, Mannitol, Croscarmellose-Natrium, Copovidon und/oder Magnesiumstearat ausgewählt ist; und der zusätzliche pharmazeutisch akzeptable Hilfsstoff, der durch das externe Zugabeverfahren zugegeben werden kann, aus mikrokristalliner Cellulose, Croscarmellose-Natrium und/oder Magnesiumstearat ausgewählt ist.

7. Kapsel, die eine Pyrrolochinolinchinon-Trilithiumsalz-Nonahydrat-Verbindung umfasst, **gekennzeichnet dadurch, dass** die Kapsel, die die Pyrrolochinolinchinon-Trilithiumsalz-Nonahydrat-Verbindung umfasst, die pharmazeutische Zusammensetzung nach einem der Ansprüche 1-6 umfasst.

8. Kapsel, die die Pyrrolochinolinchinon-Trilithiumsalz-Nonahydrat-Verbindung nach Anspruch 7 umfasst, **gekennzeichnet dadurch, dass** das Kapselmaterial aus einer Gelatinekapsel, einer Hydroxypropylmethylcellulose-Kapsel, einer Pullulan-Polysaccharid-Kapsel oder einer Stärkekapsel ausgewählt werden kann.

9. Kapsel, die die Pyrrolochinolinchinon-Trilithiumsalz-Nonahydrat-Verbindung nach Anspruch 8 umfasst, **gekennzeichnet dadurch, dass** das Kapselmaterial eine Hydroxypropylmethylcellulose-Kapsel ist.

10. Kapsel, die die Pyrrolochinolinchinon-Trilithiumsalz-Nonahydrat-Verbindung nach Anspruch 7 umfasst, **gekennzeichnet dadurch, dass** in der Kapselformulierung jede Kapsel 5 mg bis 100 mg der Pyrrolochinolinchinon-Trilithiumsalz-Nonahydrat-Verbindung umfasst.

11. Kapsel, die die Pyrrolochinolinchinon-Trilithiumsalz-Nonahydrat-Verbindung nach Anspruch 10 umfasst, **gekennzeichnet dadurch, dass** jede Kapsel 20 mg bis 75 mg der Pyrrolochinolinchinon-Trilithiumsalz-Nonahydrat-Verbindung umfasst.

12. Verfahren zur Herstellung einer Kapsel, die die Pyrrolochinolinchinon-Trilithiumsalz-Nonahydrat-Verbindung umfasst, die zur Herstellung der Kapsel verwendet wird, die die Pyrrolochinolinchinon-Trilithiumsalz-Nonahydrat-Verbindung nach Anspruch 7 umfasst, **gekennzeichnet dadurch, dass** das Herstellungsverfahren ein Trockengranulationsverfahren zur Herstellung einer Kapsel ist.

13. Verfahren zur Herstellung der Kapsel, die die Pyrrolochinolinchinon-Trilithiumsalz-Nonahydrat-Verbindung nach Anspruch 12 umfasst, **gekennzeichnet dadurch, dass**, wenn der pharmazeutisch akzeptable Hilfsstoff durch das interne Zugabeverfahren zugegeben wird, das Herstellungsverfahren Folgendes umfasst:
Abwiegen des aktiven pharmazeutischen Wirkstoffs (API) der Pyrrolochinolinchinon-Trilithiumsalz-Nonahydrat-Verbindung und des pharmazeutisch akzeptablen Hilfsstoffs gemäß den Formulierungsverhältnissen;
Mischen des API der Pyrrolochinolinchinon-Trilithiumsalz-Nonahydrat-Verbindung mit dem pharmazeutisch akzeptablen Hilfsstoff;
Ausführen einer Trockengranulation des gemischten API der Pyrrolochinolinchinon-Trilithiumsalz-Nonahydrat-Verbindung und des pharmazeutisch akzeptablen Hilfsstoffs, wobei die Mischung zunächst zu dünnen Schichten gewalzt und anschließend zu Granulat granuliert wird; und
Ausführen der Füllung der Kapseln mit dem Granulat, um die Kapsel zu erhalten.

14. Verfahren zur Herstellung der Kapsel, die die Pyrrolochinolinchinon-Trilithiumsalz-Nonahydrat-Verbindung nach Anspruch 12 umfasst, **gekennzeichnet dadurch, dass**, wenn der pharmazeutisch akzeptable Hilfsstoff durch das interne-externe Zugabeverfahren zugegeben wird, das Herstellungsverfahren Folgendes umfasst:
Abwiegen des aktiven pharmazeutischen Wirkstoffs (API) der Pyrrolochinolinchinon-Trilithiumsalz-Nonahydrat-Verbindung und des pharmazeutisch akzeptablen Hilfsstoffs gemäß den Formulierungsverhältnissen;
Mischen des API der Pyrrolochinolinchinon-Trilithiumsalz-Nonahydrat-Verbindung und des pharmazeutisch akzeptablen Hilfsstoffs für die interne Zugabe;
Ausführen einer Trockengranulation des gemischten API der Pyrrolochinolinchinon-Trilithiumsalz-Nonahydrat-Verbindung und des pharmazeutisch akzeptablen Hilfsstoffs für die interne Zugabe, wobei die Mischung zunächst zu dünnen Schichten gewalzt und anschließend zu Granulat granuliert wird;
Mischen des durch die Trockengranulation erhaltenen Granulats mit dem pharmazeutisch akzeptablen Hilfsstoff für die externe Zugabe; und
Ausführen der Füllung von Kapseln mit dem Granulat, das mit dem pharmazeutisch akzeptablen Hilfsstoff für die externe Zugabe vermischt wurde, um die Kapsel zu erhalten.

## Revendications

1. Composition pharmaceutique comprenant un composé de sel de trilithium de pyrroloquinoléine quinone nonahydraté, **caractérisée en ce que** la composition pharmaceutique comprend :
1,0 partie en poids de composé de sel de trilithium de pyrroloquinoléine quinone nonahydraté ; et un excipient pharmaceutiquement acceptable ;
l'excipient pharmaceutiquement acceptable comprend :
de 0,16 partie en poids à 0,48 partie en poids de silice colloïdale ;
où l'excipient pharmaceutiquement acceptable comprend en outre :
de 1,0 partie en poids à 5,0 parties en poids d'excipient(s) pharmaceutiquement acceptable(s) supplémentaire(s) ;
l'excipient pharmaceutiquement acceptable supplémentaire est choisi parmi un ou plusieurs des éléments suivants : amidon, amidon prégélatinisé, cellulose microcristalline, lactose, complexe lactose-amidon, complexe lactose-cellulose, mannitol, complexe mannitol-amidon, sorbitol, povidone, copovidone, hydroxypropylméthylcellulose, hydroxypropylcellulose, hydroxypropylcellulose faiblement substituée, croscarmellose sodique, crospovidone, stéarate de magnésium, stéarylfumarate de sodium, poudre de talc et acide stéarique ;
où le composé de sel de trilithium de pyrroloquinoléine quinone nonahydraté est sous une forme cristalline, et le composé de sel de trilithium de pyrroloquinoléine quinone nonahydraté présente un diagramme de diffraction des rayons X sur poudre comprenant des pics de diffraction caractéristiques à des angles de diffraction 2θ de 6,2° ± 0,2°, 7,4° ± 0,2°, 7,9° ± 0,2° et 23,6° ± 0,2°.

2. Composition pharmaceutique comprenant le composé de sel de trilithium de pyrroloquinoléine quinone nonahydraté selon la revendication 1, **caractérisée en ce que** la silice colloïdale est présente à hauteur de 0,2 partie en poids à 0,4 partie en poids ; et l'excipient pharmaceutiquement acceptable supplémentaire est présent à hauteur de 2,0 parties en poids à 4,0 parties en poids.

3. Composition pharmaceutique comprenant le composé de sel de trilithium de pyrroloquinoléine quinone nonahydraté selon la revendication 2, **caractérisée en ce que** l'excipient pharmaceutiquement acceptable supplémentaire comprend : de 0,6 partie en poids à 4 parties en poids d'amidon prégélatinisé ; de 0,36 partie en poids à 0,96 partie en poids de cellulose microcristalline ; et de 0 partie en poids à 0,06 partie en poids de stéarate de magnésium.

4. Composition pharmaceutique comprenant le composé de sel de trilithium de pyrroloquinoléine quinone nonahydraté selon la revendication 1, **caractérisée en ce que** la silice colloïdale peut être ajoutée par une méthode d'addition interne ou par une méthode d'addition interne-externe ; et l'excipient pharmaceutiquement acceptable supplémentaire peut être ajouté par une méthode d'addition interne ou par une méthode d'addition interne-externe.

5. Composition pharmaceutique comprenant le composé de sel de trilithium de pyrroloquinoléine quinone nonahydraté selon la revendication 4, **caractérisée en ce que**, lorsque la silice colloïdale est ajoutée par la méthode d'addition interne-externe, le rapport pondéral entre la silice colloïdale ajoutée en interne et la silice colloïdale ajoutée en externe est supérieur à 3:1 ; et lorsque l'excipient pharmaceutiquement acceptable supplémentaire est ajouté par la méthode d'addition interne-externe, le rapport pondéral entre l'excipient pharmaceutiquement acceptable supplémentaire ajouté en interne et l'excipient pharmaceutiquement acceptable supplémentaire ajouté en externe est supérieur à 3:1.

6. Composition pharmaceutique comprenant le composé de sel de trilithium de pyrroloquinoléine quinone nonahydraté selon la revendication 4, **caractérisée en ce que** l'excipient pharmaceutiquement acceptable supplémentaire qui peut être ajouté par la méthode d'addition interne est choisi parmi un ou plusieurs des éléments suivants : amidon, lactose, cellulose microcristalline, mannitol, croscarmellose sodique, copovidone et stéarate de magnésium ; et l'excipient pharmaceutiquement acceptable supplémentaire qui peut être ajouté par la méthode d'addition externe est choisi parmi un ou plusieurs des éléments suivants : cellulose microcristalline, croscarmellose sodique et stéarate de magnésium.

7. Capsule comprenant un composé de sel de trilithium de pyrroloquinoléine quinone nonahydraté, **caractérisée en ce que** la capsule comprenant le composé de sel de trilithium de pyrroloquinoléine quinone nonahydraté comprend la composition pharmaceutique selon l'une quelconque des revendications 1 à 6.

8. Capsule comprenant le composé de sel de trilithium de pyrroloquinoléine quinone nonahydraté selon la revendication 7, **caractérisée en ce que** le matériau de la capsule peut être choisi parmi l'un quelconque d'une capsule de gélatine, d'une capsule d'hydroxypropylméthylcellulose, d'une capsule de polysaccharide de pullulane et d'une capsule d'amidon.

9. Capsule comprenant le composé de sel de trilithium de pyrroloquinoléine quinone nonahydraté selon la revendication 8, **caractérisée en ce que** le matériau de la capsule est une capsule d'hydroxypropylméthylcellulose.

10. Capsule comprenant le composé de sel de trilithium de pyrroloquinoléine quinone nonahydraté selon la revendication 7, **caractérisée en ce que**, dans la formulation de la capsule, chaque capsule comprend de 5 mg à 100 mg de composé de sel de trilithium de pyrroloquinoléine quinone nonahydraté.

11. Capsule comprenant le composé de sel de trilithium de pyrroloquinoléine quinone nonahydraté selon la revendication 10, **caractérisée en ce que** chaque capsule comprend de 20 mg à 75 mg de composé de sel de trilithium de pyrroloquinoléine quinone nonahydraté.

12. Procédé de préparation d'une capsule comprenant le composé de sel de trilithium de pyrroloquinoléine quinone nonahydraté, qui est employé pour préparer la capsule comprenant le composé de sel de trilithium de pyrroloquinoléine quinone nonahydraté selon la revendication 7, **caractérisé en ce que** le procédé de préparation est un procédé de granulation par voie sèche pour la préparation d'une capsule.

13. Procédé de préparation de la capsule comprenant le composé de sel de trilithium de pyrroloquinoléine quinone nonahydraté selon la revendication 12, **caractérisé en ce que**, lorsque l'excipient pharmaceutiquement acceptable est ajouté par la méthode d'addition interne, le procédé de préparation comprend :
le pesage du principe actif (API) du composé de sel de trilithium de pyrroloquinoléine quinone nonahydraté et de l'excipient pharmaceutiquement acceptable selon les rapports de formulation ;
le mélange de l'API du composé de sel de trilithium de pyrroloquinoléine quinone nonahydraté avec l'excipient pharmaceutiquement acceptable ;
la réalisation d'une granulation par voie sèche sur le mélange de l'API du composé de sel de trilithium de pyrroloquinoléine quinone nonahydraté et de l'excipient pharmaceutiquement acceptable, en laminant d'abord le mélange sous forme de feuilles minces, puis en le granulant sous forme de granulés ; et
la réalisation d'un remplissage de capsule avec les granulés pour obtenir la capsule.

14. Procédé de préparation de la capsule comprenant le composé de sel de trilithium de pyrroloquinoléine quinone nonahydraté selon la revendication 12, **caractérisé en ce que**, lorsque l'excipient pharmaceutiquement acceptable est ajouté par la méthode d'addition interne-externe, le procédé de préparation comprend :
le pesage du principe actif (API) du composé de sel de trilithium de pyrroloquinoléine quinone nonahydraté et de l'excipient pharmaceutiquement acceptable selon les rapports de formulation ;
le mélange de l'API du composé de sel de trilithium de pyrroloquinoléine quinone nonahydraté et de l'excipient pharmaceutiquement acceptable pour addition interne ;
la réalisation d'une granulation par voie sèche sur le mélange de l'API du composé de sel de trilithium de pyrroloquinoléine quinone nonahydraté et de l'excipient pharmaceutiquement acceptable pour addition interne, en laminant d'abord le mélange sous forme de feuilles minces, puis en le granulant sous forme de granulés ;
le mélange des granulés obtenus par la granulation par voie sèche avec l'excipient pharmaceutiquement acceptable pour addition externe ; et
la réalisation d'un remplissage de capsule avec les granulés mélangés avec l'excipient pharmaceutiquement acceptable pour addition externe, pour obtenir la capsule.
